Europäisches Patentamt

European Patent Office

Office européen des brevets

(19)

(11) Veröffentlichungsnummer: **0 219 006**
**A2**

(12) # EUROPÄISCHE PATENTANMELDUNG

(21) Anmeldenummer: **86113628.1**

(22) Anmeldetag: **02.10.86**

(51) Int. Cl.⁴: **C 12 N 9/04**
**C 12 Q 1/60**
**//(C12N9/04, C12R1:465)**

(30) Priorität: **15.10.85 DE 3536689**

(43) Veröffentlichungstag der Anmeldung:
**22.04.87 Patentblatt 87/17**

(84) Benannte Vertragsstaaten:
**AT BE CH DE ES FR GB GR IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Betz, Joachim, Dr.**
**Hohe Stätte 16**
**D-6000 Frankfurt am Main 56(DE)**

(72) Erfinder: **Poetsch, Matthias, Dr.**
**Tiroler Strasse 14**
**D-8190 Wolfratshausen(DE)**

(54) **Verfahren zur Herstellung von Cholesterinoxidase durch Fermentation von Streptomyces hydrogenans.**

(57) Cholesterinoxidase kann durch Fermentation von Streptomyces hyrogenans DSM 3854 synthetisiert werden. Das Bakterium wird aerob in einem Nahrmedium, das Kohlenstoff- und Stickstoffquellen enthält, kultiviert, bis sich Cholesterinoxidase anhäuft.

EP 0 219 006 A2

Croydon Printing Company Ltd.

Verfahren zur Herstellung von Cholesterinoxidase durch Fermentation von Streptomyces hydrogenans

Unter Cholesterinoxidasen versteht man Enzyme oder Enzymgemische, die die 3-OH-Gruppe des Cholesterins in eine 3-Keto-Gruppe umwandeln. Oxidationen mit diesen Enzymen sind in einer Vielzahl von Patenten und Patentanmeldungen beschrieben. Die folgende Liste stellt eine Auswahl dar: FR 2 417 228, EP 21 311, DE 2 650 753, DE 2 656 063, US 4 043 870, JP 54/138 188, JP 54/113 493, JP 54/76 893, JP 54/1 105 291. Brevibacterium sterolicum, Arthrobacter simplex B 7, Nocardia erythropolis, Schizophyllum commune und Streptomyces lavendulae sind u.a. als Cholesterinoxidase-Produzenten beschrieben. Das Enzym hat für die klinische Bestimmung von Cholesterin in Körperflüssigkeiten große Bedeutung.

Es wurde nun gefunden, daß Streptomyces hydrogenans, der bei der DSM unter der Nummer 3854 hinterlegt wurde, ebenfalls eine Cholesterinoxidase synthetisiert.

Die Erfindung betrifft somit ein Verfahren zur Herstellung von Cholesterinoxidase, das dadurch gekennzeichnet ist, daß Streptomyces hydrogenans DSM 3854 aerob in einem Nährmedium, das Kohlenstoff- und Stickstoffquellen enthält, kultiviert wird, bis sich Cholesterinoxidase anhäuft.

Streptomyces hydrogenans wird in der Deutschen Auslegungsschrift 1 016 263 beschrieben. Zur Cholesterinoxidase-Produktion wird Streptomyces hydrogenans DSM 3854 durch aerobe Kultivierung bei 22 bis 30°C, vorzugsweise bei 25 bis 28°C, in einem Nährmedium gezüchtet, das Kohlenstoffquellen und Stickstoffquellen als Hauptbestandteile enthält. Als Kohlenstoffquelle werden bevorzugt 0,1 bis 2%, insbesondere 0,5 bis 1%, Kohlenhydrate eingesetzt, bezogen auf das Gewicht des Nährmediums. Stickstoffquellen sind bevorzugt organische Stickstoffverbindungen, die der Nährlösung in Mengen von 1 bis 10 Gew.-%, vorzugsweise 2 bis 5 Gew.-%, zugesetzt werden. Der Nährlösung können weiterhin noch anorganische Salze zugegeben werden, beispielsweise Ammoniumsulfat und Magnesiumsulfat.

Als Inokulum dient eine Sporensuspension von Streptomyces hydrogenans DSM 3854 bzw. eine Vorkultur, die 1 bis 5 Gew.-% Biomasse enthält. Es ist vorteilhaft, das Bakterium in Submerskultur über einen Zeitraum von 1 bis 5 Tagen zu inkubieren. Ein Zusatz von Cholesterin zur Nährlösung wirkt induktiv und erhöht die Ausbeute an Cholesterinoxidase. Die Zugabe von bis zu 1% eines Antischaummittels verhindert eine zu starke Schaumbildung während der Fermentation.

Cholesterinoxidase kann nach der Fermentation unter Anwendung von bekannten protein-chemischen Verfahren aus der Kulturlösung isoliert werden und durch Gefriertrocknung, als Lösung in Glycerin oder als Kristallsuspension haltbar gemacht werden.

Die Konzentration der erfindungsgemäßen Cholesterinoxidase in der Kulturlösung wird mit Hilfe der Oxidationsreaktion des Cholesterins zu Cholestenon bestimmt. Das Enzym eignet sich dementsprechend auch hervorragend zur klinischen Bestimmung von Cholesterin in Körperflüssigkeiten.

Die folgenden Beispiele stellen eine detaillierte Beschreibung der Erfindung dar. Prozentangaben beziehen sich, wenn nicht anders angegeben, auf das Gewicht.
Beispiel 1

Streptomyces hydrogenans DSM 3854 wird in Schrägröhrchen auf einem Nährboden folgender Zusammensetzung gezüchtet.

1,5 % Agar

3 % Erdnußmehl (Fa. K.u.K. Chemie, Hamburg)

2 % Maisstärke (Fa. Roquette GmbH, Frankfurt)

0,5 % Ammoniumsulfat

0,8 % Kalziumkarbonat

1 % Cornsteep fest (Fa. Roquette GmbH, Frankfurt)

4 % Dextrin weiß (Fa. Roquette GmbH; Frankfurt)

0,5 % Magnesiumsulfat·7 $H_2O$

Nach einer Inkubationszeit von 10 Tagen bei 28°C werden die Sporen mit 3 ml physiologischer Kochsalzlösung abgeschwemmt und jeweils 1 ml dieser Suspension zum Animpfen von 100 ml Vorkultur der obengenannten Zusammensetzung, ohne den Zusatz von Agar, verwendet.

Die Impfkolben werden 30 h bei 28°C auf einer Schüttelmaschine bei 230 Upm inkubiert. Dann werden je 50 ml dieser Vorkultur in 2 l fassende Erlenmeyerkolben mit je 500 ml der genannten Nährlösung gebracht und als Hauptkultur 3 Tage bei 28°C geschüttelt (230 Upm). Die Zellen werden von der Kulturlösung abgetrennt.

Beispiel 2

Ein nach Beispiel 1 erhaltenes Kulturfiltrat der Hauptkultur wird mit Cholesterin inkubiert, so daß die Endkonzentration im Ansatz nach der Inkubation ungefähr 0,025 mg/ml beträgt. Über einen Zeitraum von 60 Minuten werden in regelmäßigen Abständen von 10 Minuten Aliquote entnommen, die mit der 10-fachen Menge ethanolischer KOH (2 ml 12 M KOH + 48 ml Ethanol) versetzt werden und 2 Minuten in einem kochenden Wasserbad gehalten werden. Es wird 1:10 (vol) mit Phosphatpuffer (50 mM, pH 7.0) verdünnt und das gebildete Cholestenon sowie das Ausgangsprodukt Cholesterin mit der 2,5-fachen Menge Isooctan extrahiert. Die optische Dichte der Isooctanschicht bei 232 nm ist der Menge an Cholestenon direkt proportional, die über den molaren Extraktionskoeffizienten von 16800-17600 oder aus einer Eichkurve bestimmt wird.

Die Aktivität des Kulturfiltrats beträgt durchschnittlich
0.1 U pro ml. Eine Einheit (U) entspricht der Cholesterinoxidasemenge, die bei pH 7,0 und 25°C 1 µM Cholesterin zu
Cholestenon oxidiert.

1. Verfahren zur Herstellung von Cholesterinoxidase durch Fermentation von Streptomyceten, dadurch gekennzeichnet, daß Streptomyces hydrogenans DSM 3854 aerob in einem Nährmedium, das Kohlenstoff- und Stickstoffquellen enthält, kultiviert wird, bis sich Cholesterinoxidase anhäuft.

2. Verfahren nach Anspruch 1, dadurch gekennzeichnet, daß die Fermentation bei 22 bis 35°C durchgeführt wird.

3. Verfahren nach Anspruch 2, dadurch gekennzeichnet, daß die Fermentation bei 25 bis 30°C durchgeführt wird.

4. Verfahren nach einem der Ansprüche 1 bis 3, dadurch gekennzeichnet, daß der Nährlösung anorganische Salze zugegeben werden.

5. Verfahren nach einem der Ansprüche 1 bis 4, dadurch gekennzeichnet, daß dem Nährmedium Cholesterin zugegeben wird.

6. Verfahren nach einem der Ansprüche 1 bis 5, dadurch gekennzeichnet, daß Streptomyces hydrogenans DSM 3854 in einer Submerskultur gezüchtet wird.

7. Cholesterinoxidase, erhältlich durch Fermentation von Streptomyces hydrogenans DSM 3854 .

8. Verwendung der Cholesterinoxidase gemäß Anspruch 7 zur Bestimmung von Cholesterin in Körperflüssigkeiten.

9. Streptomyces hydrogenans DSM 3854 und seine Mutanten und Varianten, sofern sie Cholesterinoxidase synthetisieren.

Patentansprüche Österreich und Spanien:

1. Verfahren zur Herstellung von Cholesterinoxidase durch Fermentation von Streptomyceten, dadurch gekennzeichnet, daß Streptomyces hydrogenans DSM 3854 aerob in einem Nährmedium, das Kohlenstoff- und Stickstoffquellen enthält, kultiviert wird, bis sich Cholesterinoxidase anhäuft.

2. Verfahren nach Anspruch 1, dadurch gekennzeichnet, daß die Fermentation bei 22 bis 35°C durchgeführt wird.

3. Verfahren nach Anspruch 2, dadurch gekennzeichnet, daß die Fermentation bei 25 bis 30°C durchgeführt wird.

4. Verfahren nach einem der Ansprüche 1 bis 3, dadurch gekennzeichnet, daß der Nährlösung anorganische Salze zugegeben werden.

5. Verfahren nach einem der Ansprüche 1 bis 4, dadurch gekennzeichnet, daß dem Nährmedium Cholesterin zugegeben wird.

6. Verfahren nach einem der Ansprüche 1 bis 5, dadurch gekennzeichnet, daß Streptomyces hydrogenans DSM 3854 in einer Submerskultur gezüchtet wird.

# ERKLÄRUNG NACH REGEL 28 ABSATZ 4 DES EUROPÄISCHEN PATENTÜBEREINKOMMENS

Der Anmelder hat dem Europäischen Patentamt mitgeteilt, daß der in Regel 28 Absatz 3 des Europäischen Patentübereinkommens vorgesehene Zugang zu den nachstehend gekennzeichneten Mikroorganismen bis zu dem Tag, an dem der Hinweis auf die Erteilung des europäischen Patents bekanntgemacht wird oder an dem die Patentanmeldung zurückgewiesen oder zurückgenommen wird oder als zurückgenommen gilt, nur durch die Herausgabe einer Probe an einen Sachverständigen hergestellt wird.

## KENNZEICHNUNG DER MIKROORGANISMEN

Eingangsnummern der Hinterlegungen: DSM 3854

EPA Form 1165 07.81 d